**Europäisches Patentamt**

**European Patent Office**

**Office européen des brevets**

(19)

(11) **EP 1 443 325 A1**

(12) **EUROPEAN PATENT APPLICATION**

(43) Date of publication:
**04.08.2004 Bulletin 2004/32**

(51) Int Cl.⁷: **G01N 33/49**, G01N 33/86

(21) Application number: **03002254.5**

(22) Date of filing: **01.02.2003**

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(71) Applicants:
• **Roche Diagnostics GmbH**
**68298 Mannheim (DE)**
Designated Contracting States:
**DE**
• **F. HOFFMANN-LA ROCHE AG**
**4070 Basel (CH)**
Designated Contracting States:
**AT BE BG CH CY CZ DK EE ES FI FR GB GR HU IE IT LU MC NL PT SE SI SK TR**

(72) Inventor: **Hill, James**
**69242 Mühlhausen (DE)**

(74) Representative: **Pfeifer, Hans-Peter, Dr. et al**
**Patentanwälte**
**Dr. H.-P. Pfeifer Dr. P. Jany**
**Beiertheimer Allee 19**
**76137 Karlsruhe (DE)**

(54) **System and method for determining a coagulation parameter**

(57) System and Method for the determination of a coagulation parameter of a blood or plasma sample of a patient, including a reagent system (5) to be mixed with the sample, thereby forming a coagulation detection liquid, a space (4) for receiving the coagulation detection liquid, and an instrument (1) adapted for monitoring coagulation of the coagulation detection liquid, and measuring a coagulation time.

The coagulation time is determined at a non-standard temperature of the coagulation detection liquid different from a standard temperature for the respective coagulation parameter, said non-standard temperature being measured, and the coagulation parameter for the standard temperature is calculated from the coagulation time measured at said non-standard temperature, using a mathematical relationship of coagulation time versus temperature which relationship is independent of the individual patient whose blood is examined.

Fig 1

EP 1 443 325 A1

**Description**

Many medical problems relate to the coagulation of blood. In particular during treatment with anticoagulant medication, a patient's coagulation parameters fluctuate continuously. Such fluctuations can cause severe problems. For example if a patient is treated with an anticoagulant drug such as Heparin or Marcumar, it is important that his or her coagulation parameters remain within a defined range of values to avoid complications. Only in this way is it possible to reduce effectively the number of blood clots while simultaneously avoiding bleeding complications. A rapid, precise method for the continuous monitoring of blood coagulation parameters that meets all therapeutic needs is therefore required.

Currently, in particular three coagulation parameters are of medical interest, namely prothrombin time (PT), activated partial thromboplastin time (APTT) and activated clotting time (ACT). PT mainly serves for monitoring the effect of vitamin K antagonists on coagulation (which influence factors II, V, VII and X of the coagulation cascade). The PT test measures the activation of the extrinsic pathway by addition of tissue thromboplastin.

APTT is used primarily to monitor heparin therapy. The test detects factor changes in the intrinsic coagulation cascade (factors VIII, IX, XI, XII and other enzymes and factors). The test reagents for this type of test have not yet been standardized and therefore differences in the heparin sensitivity of reagents from different manufacturers are considerable.

ACT is determined to monitor heparinization in situations where an APTT test cannot be performed, because the patient was administered a high dose of heparin.

Traditionally, the coagulation parameters are determined by "wet chemistry" tests. An aliquot of blood sample is mixed with liquid reagents and the point of time at which the blood clots is detected. The results are indicated either directly (in seconds) or in the form of derived quantities such as ratio to a respective normal value (in percent). With respect to PT further common quantities for indication of the test results are % Quick and INR (International Normalized Ratio).

Since several years so-called "dry chemistry" tests of coagulation parameters have become available. They are performed by means of test systems comprising disposable reagent carrier elements (often designated "test elements") and an evaluation instrument which generally is adapted for the evaluation of a particular type of test element from a particular manufacturer. The test element contains the reagent system necessary for the particular test and, preferably, suitable information for the evaluation of the test such as the test type, the lot number and the expiration date.

After a test element is inserted into the evaluation instrument, the information coded on it is read.

Each test element is designed to allow the blood sample to first move from an application field into the reaction chamber by means of capillary forces or external pressure. The reaction chamber contains the required mixture of reagents that initiate the blood coagulation cascade. After mixing of the sample with the reagents the resulting liquid is monitored until coagulation is detected. The amount of time between sample application and onset of coagulation is measured. This time is converted into the appropriate unit of measurement for the coagulation parameter. To this end evaluation data stored in the instrument and/or in the test element can be used. The results are then displayed on a display of the instrument and/or forwarded to further evaluation, for example by a separate computer system.

Such a system is commercially available from the applicants under the trade name CoaguChek®. Further details may be found in the appropriate literature including US patent 5,789,664 and WO 01/11356.

The invention refers in particular to such modern dry chemistry tests. It is, however, not limited to this field of application. Rather it can be used with any type of test for coagulation parameters which involves mixing of a blood or plasma sample (which depending on the test may be pretreated, in particular by reaction with a preparatory anticoagulant) with a reagent system (which normally comprises a plurality of reagents) and thereafter monitoring the coagulation (preferably by optical or electro-chemical means). The sample may be whole blood (generally in dry chemistry test systems) or plasma (generally in laboratory systems). Hereafter reference is made to blood as an example. This should, however, not be understood as a limitation to the general applicability of the invention.

While tests for coagulation parameters have become relatively simple, in particular due to the introduction of dry chemistry tests, it remains an important task to further simplify the design and thus reduce the cost without sacrificing accuracy. This is in particular true for small battery-operated instruments which are used by the patients themselves in order to monitor their blood coagulation status. This so-called "home-monitoring" is of paramount medical importance for patients who require long-term medication. For example, if a person received a replacement cardiac valve his or her long-term health highly depends on the fact that the coagulation status remains reliably between certain boundaries. This can be achieved much better if the patient monitors the coagulation parameters rather than having only occasional tests performed in a clinic or a doctor's office. Similar considerations also apply to "point of care (POC)" testing by the medical profession.

Therefore the invention addresses the task to simplify the design of systems for determining coagulation parameters while simultaneously maintaining the required degree of accuracy.

This task is achieved by a system for the determination of a coagulation parameter of a blood sample of a patient, said system including a reagent system to be mixed with the blood sample, thereby forming a coagulation detection liquid, a space for receiving the coagulation detection liquid, an instrument adapted for monitoring clotting of the coagulation detection liquid, the instrument comprising an evaluation unit including a time measurement circuit for measuring a coagulation time between the mixing of the reagent system with the sample and coagulation and for deriving the desired coagulation parameter therefrom, which is characterized in that the coagulation time is determined at a non-standard temperature of the coagulation detection liquid different from a standard temperature for the respective coagulation parameter, said non-standard temperature being measured, and the coagulation parameter for the standard temperature is calculated from the coagulation time measured at said non-standard temperature, using a mathematical relationship of coagulation time versus temperature which relationship is independent of the individual patient whose blood is examined, and by a corresponding method.

Known systems for the determination of coagulation parameters always comprise some kind of thermostating device designed to maintain during the coagulation measurement a defined standard temperature, usually 37°C. This requires an electric heater and an electronic temperature control system. Based on the invention such a thermostating device is not necessary. Rather the coagulation measurement can be made at a convenient temperature (e.g. room temperature). Nevertheless accurate values of the desired coagulation parameter can be determined which are directly comparable to those determined at the standard temperature. This leads to a plurality of advantages:

- For small portable battery operated coagulation instruments the heating system is expensive and consumes more battery energy than all other components combined. The volume, weight and cost of the instruments can be reduced substantially if no heating system and a smaller battery are required.

- In order to secure an exact control of the desired standard temperature test elements of known dry chemistry systems often have a long capillary for guiding the sample liquid from the point of sample application (outside the instrument) into the interior of the instrument where the thermostating device is located. If no such long capillary is necessary the sample volume can be reduced.

- The warm up time required with known instruments before testing is eliminated or reduced dramatically. This simplifies the use of the system, in particular in home monitoring or POC applications.

- For laboratory systems inaccuracies associated with overincubating the sample and/or thromboplastin reagent are reduced by using lower temperatures.

- Also for laboratory systems the final individual instrument reaction temperature may vary due to limitations of the thermostating quality. This can be corrected to equal standard temperatures.

- For INR calibration a manual technique is generally used which causes difficulties in controlling the standard (37°C) temperature. Errors due to temperature deviations can be reduced if the technique can be performed at room temperature.

During the experimental work on which the invention is based it has been determined that the change of coagulation time parameters versus temperature can be described by a functional relationship which is specific for a particular instrument and a particular reagent system but surprisingly is independent on the sample. Therefore the same functional relationship f(T) (which may e.g. be a linear function of a polynomial) describes the temperature dependence no matter whose blood is being examined or what the coagulation status of the particular patient (depending e.g. on Marcumar dosage) is. Taking PT as an example this may be expressed mathematically:

$$(1) \qquad PT^{37} = f(T) * PT^{T}$$

where

$PT^{37}$: PT value for the standard temperature of 37°C

$PT^{T}$: PT value for a (lower) non-standard temperature.

Generally in the field of clinical chemistry it is not unusual to perform a temperature correction calculation if a test is temperature dependent and the actual test temperature differs from a desired standard test temperature. For example tests for determining the concentration of glucose in blood are temperature dependent and it has been proposed to eliminate errors caused by temperature variations by an appropriate correction calculation (see e.g. US patents 5,405,511 and 5,972,715).

Coagulation detection is, however, fundamentally different from the detection of the concentration of an analyte such as glucose. The temperature dependence of glucose tests is caused by influencing factors which are independent on the individual sample which is tested. In particular the enzymatic reaction on which the test is based depends on the temperature. A coagulation test is fundamentally different since it is essentially an experimental model of the natural coagulation process which - as is well known - involves a highly complicated reaction of more than ten factors and enzymes in the blood of the particular patient. The fact that the coagulation behaviour of blood is different for every individual is also apparent from the fact that the dosage of corresponding medication (e.g. Heparin or Marcumar) has to be individually adapted.

Therefore in coagulation testing the quantity which is measured depends on the particular individual sample, i.e. the individual whose blood is tested and the status of his or her coagulation system. Regarding the temperature dependence the same had to be assumed. Therefore it could not be expected that, by examining the blood of a limited number of patients at different temperatures with the same test system (same type of instrument and same reagent system, preferably of the same manufacturing lot) a universal f(T)-curve could be generated which allows calculation of $PT^{37}$ from $PT^T$ values determined at a non-standard temperature for additional patients with quite different blood.

In the prior art PT and APTT tests have not purposely been performed at temperatures outside the standard range at about 37°C. An exception is described in

A. Uldall: "Prothrombin time standardization and temperature problems", Clinica Chimica Acta, 1980, 39-44.

This publication refers to PT tests performed in a glass tube which are immersed into a water bath to allow exact control of the temperature. The author refers to problems caused by deviations from the standard temperature of 37°C, e.g. in case the glass tube is not immersed sufficiently deeply and by the fact that the tube has to be withdrawn from the water bath repeatedly in order to check the coagulation status. In order to reduce these problems a lowering of the generally accepted standard temperature from 37°C to 30°C is considered.

US-patent 5,031,619 refers to a Bleeding Time Test. In such tests an incision of predetermined dimensions is applied to the skin of a patient and the time is measured from a moment that bleeding through the incision begins to the moment coagulation appears to occur. Such bleeding time measurements are used as screening tests for evaluation of the hemostatic adequacy of platelets which depends on platelet number and platelet function. It is a qualitative (or at most semi-quantitative) test allowing screening for certain disorders (e. g. Glanzmann's thrombasthenia). The patent refers to the earlier known fact that the rate at which external bleeding occurs is roughly inversely proportional to the temperature of the subject's skin and suggests to use a fixed factor of 0.05 per degree in order to compensate for variations of the temperature of the subject.

The invention will be further described hereafter with reference to the figures wherein

Fig. 1        shows a highly schematic diagram of a coagulation test system;

Fig. 2        shows a graphical representation of the ratio $PT^{37}/PT^T$ vs. T[°C]/37 for a series of experiments performed in the context of the invention;

Fig. 3        shows a graphical representation of experimental results comparing the invention with a conventional determination of INR values;

Fig. 4 to 8   show results corresponding to figure 2 performed with different coagulation parameter tests.

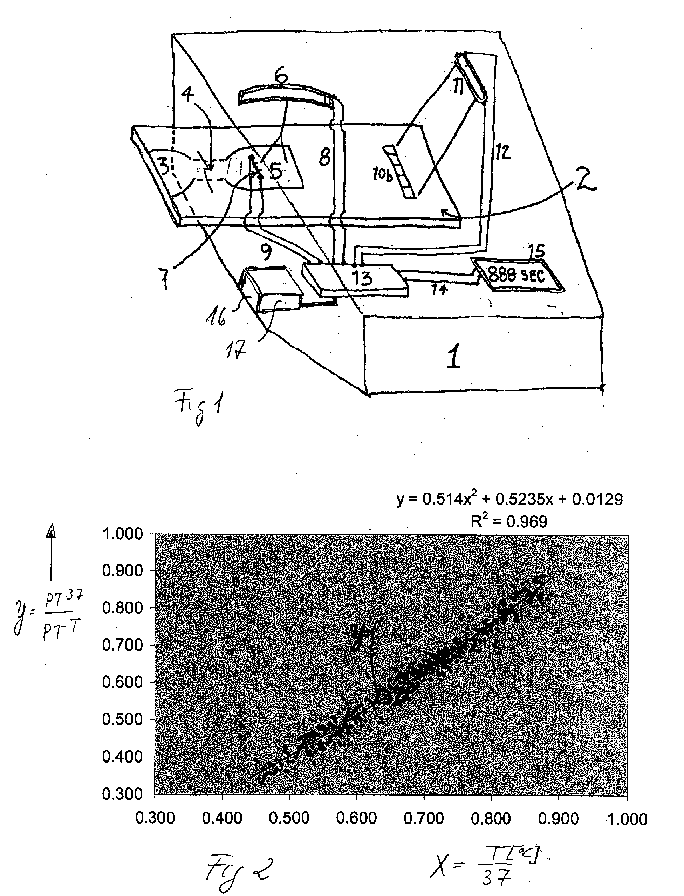

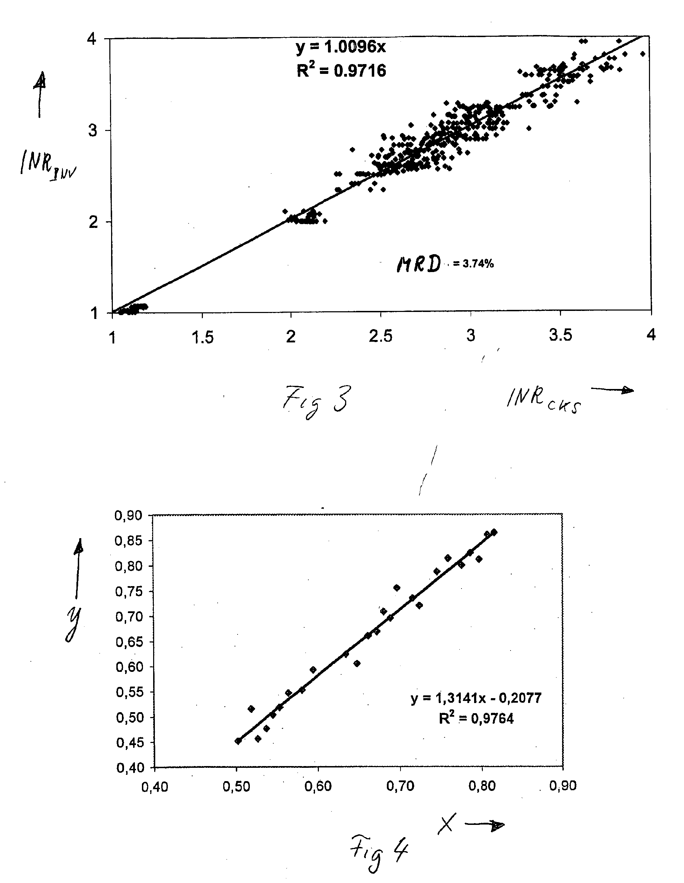

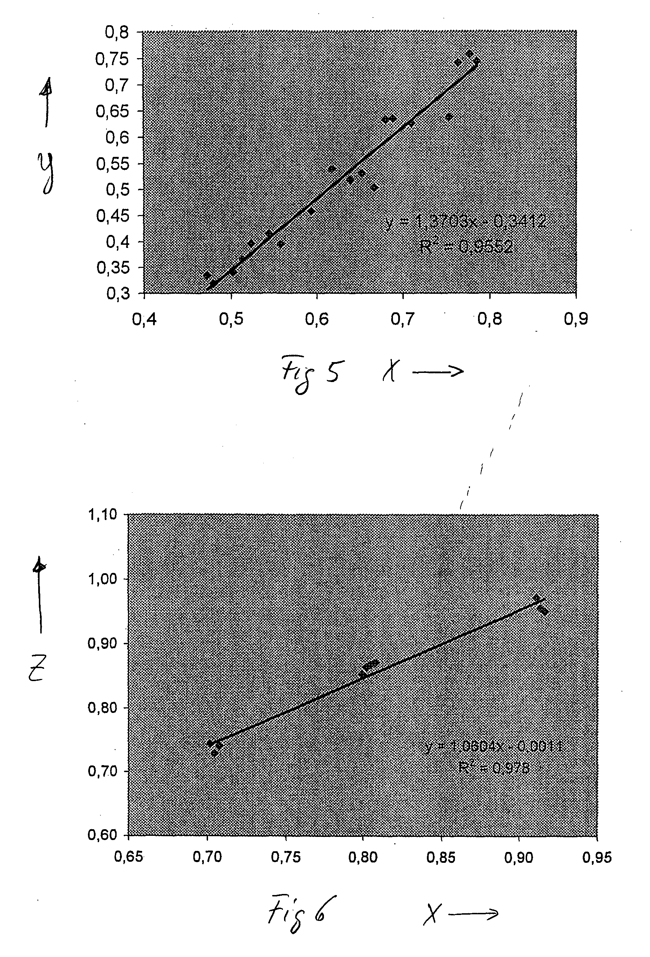

Figure 1 is a schematic representation of a coagulation test system comprising an instrument 1 and a disposable test element 2. The test element has a sample application field 3, a capillary channel 4 and a space 5 which serves as reaction chamber and coagulation detection area. The channel 4 is very short and can even be omitted because, as mentioned above, it is no longer needed for temperature control reasons. Space 5 receives the coagulation detection liquid which is formed by mixing of a sample applied to the sample application field 3 and a reagent system (not shown).

A coagulation detection device 6 is located adjacent to the coagulation detection area for detection of coagulation therein. The resulting signals are transmitted via lines 8 to a measurement and evaluation unit 13 which controls the operation of the instrument. Coagulation detection can be performed by any of the methods known from the prior art, in particular by optical or electro-chemical means, see e.g. US patent 5,789,664 and WO 01/11356.

A temperature measuring device 7 (e.g. a thermocouple or thermistor) is located in close proximity or even inside space 5 containing the reaction liquid during coagulation detection. For direct temperature detection the temperature measurement device can be integrated into the test element. Its signals are delivered via lines 9 to measurement and evaluation unit 13. The required electrical connection to a thermocouple or other temperature measurement device integrated into the test element can be established by plug-in contacts (not shown) as is customary for electrochemical test elements.

The test element carries information relating to test type, reagent lot and possibly also process information in an information field 10 which is evaluated by an element information detection device 11. This information may in particular include calibration data required for evaluation of the test. These data may depend on the manufacturing lot of the test element. The detected information signal is transmitted via lines 12 to the measurement and evaluation unit 13.

A ROM key 16 sitting exchangeably in a ROM key holder 17 may be provided as an additional or alternative machine readable code carrier element and is also connected to the central measurement and evaluation unit 13 for data exchange therewith.

The information carried by the test element may be stored in any known machine readable form such as a barcode, a magnetic strip or an integrated solid state memory chip. In a particular preferred embodiment it also includes data describing the mathematical relationship of coagulation time versus temperature which is used for calculating the desired coagulation parameter for a standard temperature from the coagulation time measured at a non-standard temperature.

These and other data may alternatively or additionally be stored in a dedicated machine readable code carrier element such a ROM key. This technology is - for electrochemical analysis systems - described in many prior art publications including for example US patent 5,366,609.

The central measurement and evaluation unit 13 combines the signal information received from coagulation detection device 6, element information device 11 and temperature measuring device 7 to derive the desired coagulation parameter. This result is transmitted via lines 14 to a display 15 of the instrument 1.

Most parts of the system shown in figure 1 are conventional and no further details need to be described. Deviating from the prior art, however, the instrument 1 must not be equipped with a thermostating system including a heater and electric heating control. Rather it only has the temperature measuring device 7 for measuring (directly or indirectly) the temperature of the coagulation detection liquid in the coagulation detection area. During experimental evaluation'of the invention it has been adequately demonstrated that measuring the temperature adjacent to the space in which the coagulation detection liquid,is contained is sufficient to allow accurate calculation of a desired standard coagulation parameter (which would have been obtained at a controlled temperature of approximately 37°C) from measurements performed at a deviating (generally substantially lower) temperature. The measurement and evaluation unit 13 is adapted to perform the required calculations.

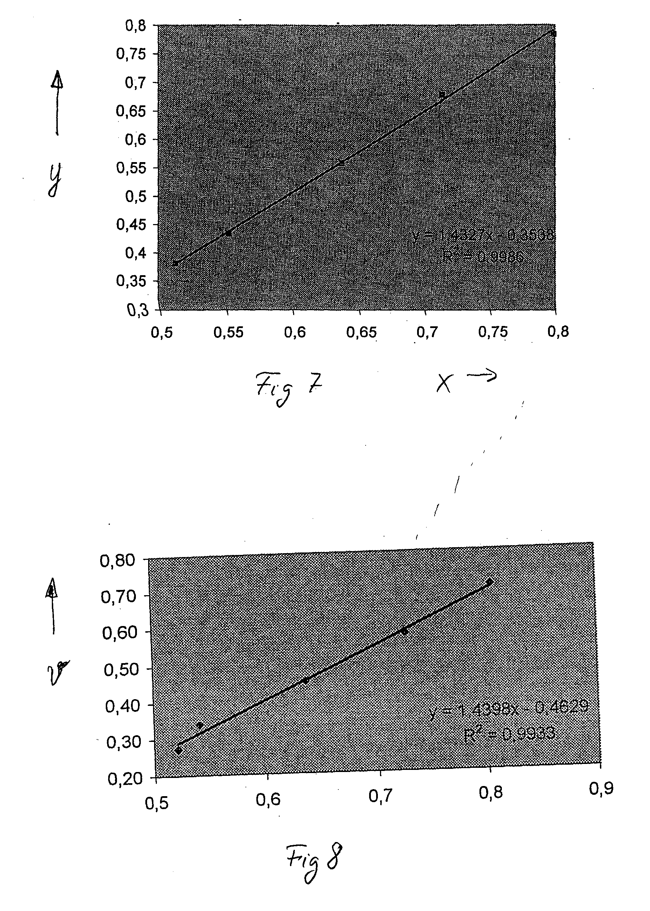

Figure 2 shows experimental results which were generated as follows:

- Samples from 27 different patients and four standard liquids ("normals") were taken, each separated in multiple aliquots.

- From each sample four measurements of $PT^T$-values were made at four non-standard temperatures between 16° and 32°C (using four aliquots). These measurements were performed with a Coagu-Check®S instrument as manufactured by the applicant which was modified by disabling its thermostating system. Temperature variation was provided by positioning the instrument in a temperature chamber.

- In order to improve the precision and increase the data base each of these measurements was performed with four different instruments.

- Simultaneously for each sample $PT^{37}$ was determined using a conventional CoaguCheck®S system with thermostating.

From the results PT-ratios $Y=PT^{37}/PT^T$ were calculated. These PT-ratios are shown in figure 2 relative to a temperature ratio X calculated by dividing the respective measurement temperature T (in °C) by 37.

Figure 2 shows that all data are very close to a curve $Y=f(X)$ determined by regression analysis therefrom. In this case the curve is a second order polynomial as shown in figure 2. This prooves the surprising fact that the temperature dependence can be described by a single functional relationship independent on the source of the samples used.

With respect to the temperature it was convenient during the practical experiments to use temperature ratios (as shown in place of the absolute temperatures). Evidently $Y=f(X)$ can be easily transformed to $Y=PT^{37}/PT^T=f(T)$. Furthermore an equation for calculating $PT^{37}$ is easily found by solving this equation for $PT^{37}$:

$$PT^{37} = f(T) * PT^T$$

Figure 3 shows a comparison of INR-values determined according to the invention (designated $INR_{INV}$) with corresponding data determined by a reference method ($INR_{CKS}$). INR values are calculated by forming a ratio between PT and a Median Normal PT (MNPT) as is well known in the art. The figure shows that the results generated by both methods are in excellent agreement with a Mean Relative Deviation (MRD) of 3,74%. It should be noted that the values of $INR_{INV}$ were determined with ambient temperature measurements between about 16°C and 32°C whereas the reference values $INR_{CKS}$ were determined at the standard temperature of 37°C.

Figures 4 to 8 show the results of experiments performed with a smaller number of samples but a plurality of different methods. In each case a ratio of a coagulation parameter at a standard temperature and the same parameter at the respective non-standard temperature (designated Y for PT-ratios, Z for INR-ratios and V for APTT-ratios) is plotted against the temperature ratio X. The individual figures are based on experiments using the following coagulation test systems:

Figure 4:

CoaguCheck® S Low ISI PT Strips. These tests use dry Low ISI thromboplastin derived from human recombinant tissue factor requiring non-anticoagulated fresh venous of capillary blood. The samples were from patients on oral anticoagulant treatment.

Figure 5:

CoaguCheck® S Low Volume PT Strips. These strips contain High ISI thromboplastin derived from rabbit brains requiring non-anticoagulated fresh venous or capillary blood.

Figure 6:

Amelung 4 Channel Lab Analyzer with Low ISI Lab reagent. This reagent contains Ortho Recombiplastin PT derived from human recombinant tissue factor requiring citrated plasma.

Figure 7:

Amelung 4 Channel Lab Analyzer with High ISI Lab reagent. This reagent includes Dade C Plus PT derived from rabbit brains requiring citrated plasma.

Figure 8:

Amelung 4 Channel Lab Analyzer with Ortho Auto APTT Lab reagent requiring citrated plasma.
In each case the resulting data can be described for all samples by a single functional relationship as shown in the figures. Thus the invention is applicable to different types of coagulation tests including both "dry chemistry" and "wet chemistry" test.


**Claims**

1. System for the determination of a coagulation parameter of a blood or plasma sample of a patient, said system including
   a reagent system to be mixed with the sample, thereby forming a coagulation detection liquid,
   a space (5) for receiving the coagulation detection liquid,
   an instrument (1) adapted for monitoring coagulation of the coagulation detection liquid,
   the instrument comprising an evaluation unit (13) including a time measurement circuit for measuring a coagulation time and for deriving the desired coagulation parameter therefrom,
   **characterized in that**
   the coagulation time is determined at a non-standard temperature of the coagulation detection liquid different from a standard temperature for the respective coagulation parameter, said non-standard temperature being measured, and
   the coagulation parameter for the standard temperature is calculated from the coagulation time measured at said non-standard temperature, using a mathematical relationship of coagulation time versus temperature which relationship is independent of the individual patient whose blood is examined.

2. System according to claim 1, **characterized in that** the instrument contains no device for thermostating the coagulation detection liquid, whereby the temperature of the coagulation detection liquid depends on the temperature of the environment in which the measurement is performed.

3. System according to any one of the preceding claims, **characterized in that** the instrument is battery-operated.

4. System according to any one of the preceding claims, **characterized in that** the reagent system and the space for receiving the coagulation detection liquid are contained in a disposable test element which is adapted to be inserted into a holder of the instrument.

5. System according to any one of the preceding claims, **characterized in that** it comprises a machine readable code carrier element, in particular a ROM key, containing data which are required for deriving the desired coagulation parameter from a measured coagulation time value.

6. System according to claim 5 **characterized in that** the code carrier element contains data describing the mathematical relationship of coagulation time versus temperature.

7. Disposable test element adapted for a system according to claim 4.

8. Test element according to claim 7 **characterized in that** it comprises a machine readable code containing calibration data which are specific for the manufacturing lot of the test element and are used for deriving the desired coagulation parameter from a measured coagulation time.

9. Test element according to any one of claims 7 or 8 **characterized in that** it comprises a machine readable code containing data describing the mathematical relationship of coagulation time versus temperature.

10. Test element according to any one of claims 6 to 9 **characterized in that** it comprises a temperature measurement device, in particular a thermocouple or a thermistor, and plug-in contacts allowing transfer of temperature signals of the temperature measurement device to an instrument into which it is inserted.

11. Method for the determination of a coagulation parameter of a blood or plasma sample of a patient, wherein

a reagent system is mixed with the sample to form a coagulation detection liquid,

the coagulation detection liquid is monitored to detect coagulation thereof,

the coagulation time is measured and a desired coagulation parameter is derived therefrom, **characterized in that**

the coagulation time is determined at a non-standard temperature of the coagulation detection liquid different from a standard temperature for the respective coagulation parameter, said non-standard temperature being measured, and

the coagulation parameter for the standard temperature is calculated from the coagulation time measured at said non-standard temperature, using a mathematical relationship of coagulation time versus temperature which relationship is independent of the individual patient whose blood is examined.

12. System according to claim 1 or method according to claim 10, **characterized in that** the same mathematical relationship is used for all determinations of a given coagulation parameter performed with the instrument, independent of the reagent lot.

13. System according to claim 1 or method according to claim 10, **characterized in that** the mathematical relationship is determined by measuring the coagulation time for a plurality of samples at different temperatures using the same type of instrument and reagents.

Fig 1

$$y = 0.514x^2 + 0.5235x + 0.0129$$
$$R^2 = 0.969$$

$$y = \frac{PT^{37}}{PT^{T}}$$

Fig 2

$$X = \frac{T[°C]}{37}$$

$$y = 1.0096x$$
$$R^2 = 0.9716$$

$INR_{INV}$

$MRD = 3.74\%$

$INR_{CKS}$

Fig 3

$$y = 1,3141x - 0,2077$$
$$R^2 = 0,9764$$

$y$

$X$

Fig 4

Fig 5 X →

Fig 6 X →

y = 1,4327x - 0,3508
R² = 0,9986

Fig 7     X →

y = 1,4398x - 0,4629
R² = 0,9933

Fig 8

**European Patent Office**

**EUROPEAN SEARCH REPORT**

Application Number

EP 03 00 2254

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | US 5 789 644 A (THELEN HANS-GUENTER ET AL) 4 August 1998 (1998-08-04) | 1,3-5,7,8 | G01N33/49 G01N33/86 |
| Y | * column 5, line 11 - column 6, line 30 * | 2,6, 10-13 | |
| Y | US 5 580 744 A (ZWEIG STEPHEN E) 3 December 1996 (1996-12-03) * column 1, line 56 - line 59 * * column 12, line 12 - line 29 * | 2,6, 10-13 | |
| A | US 6 066 504 A (JINA ARVIND N) 23 May 2000 (2000-05-23) * column 8, line 8 - line 40 * | 4-9 | |
| A | US 6 150 174 A (ANDERSON CARTER R ET AL) 21 November 2000 (2000-11-21) * column 2, line 48 - column 3, line 13 * | 1,2,4,7, 11 | |

TECHNICAL FIELDS
SEARCHED     (Int.Cl.7)

G01N

The present search report has been drawn up for all claims

| Place of search | Date of completion of the search | Examiner |
|---|---|---|
| THE HAGUE | 13 August 2003 | Wilhelm, J |

EPO FORM 1503 03.82 (P04C01)

## ANNEX TO THE EUROPEAN SEARCH REPORT
## ON EUROPEAN PATENT APPLICATION NO.

EP 03 00 2254

This annex lists the patent family members relating to the patent documents cited in the above-mentioned European search report.
The members are as contained in the European Patent Office EDP file on
The European Patent Office is in no way liable for these particulars which are merely given for the purpose of information.

13-08-2003

| Patent document cited in search report | | Publication date | Patent family member(s) | | Publication date |
|---|---|---|---|---|---|
| US 5789644 | A | 04-08-1998 | DE | 4422815 A1 | 04-01-1996 |
| | | | CA | 2152222 A1 | 30-12-1995 |
| | | | CN | 1126193 A ,B | 10-07-1996 |
| | | | DE | 59500837 D1 | 27-11-1997 |
| | | | EP | 0690036 A1 | 03-01-1996 |
| | | | NO | 952602 A | 02-01-1996 |
| US 5580744 | A | 03-12-1996 | US | 5418143 A | 23-05-1995 |
| | | | AU | 4287593 A | 29-11-1993 |
| | | | CA | 2133983 A1 | 11-11-1993 |
| | | | DE | 69329280 D1 | 28-09-2000 |
| | | | DE | 69329280 T2 | 15-03-2001 |
| | | | DK | 638127 T3 | 18-12-2000 |
| | | | EP | 0638127 A1 | 15-02-1995 |
| | | | ES | 2151509 T3 | 01-01-2001 |
| | | | GR | 3034647 T3 | 31-01-2001 |
| | | | JP | 3368279 B2 | 20-01-2003 |
| | | | JP | 7506429 T | 13-07-1995 |
| | | | WO | 9322453 A1 | 11-11-1993 |
| US 6066504 | A | 23-05-2000 | US | 6046051 A | 04-04-2000 |
| | | | US | 6060323 A | 09-05-2000 |
| | | | US | 6338821 B1 | 15-01-2002 |
| US 6150174 | A | 21-11-2000 | AU | 6681298 A | 22-09-1998 |
| | | | WO | 9839643 A1 | 11-09-1998 |